(19) Europäisches Patentamt
European Patent Office
Office européen des brevets

(11) Publication number: **0 170 470 A2**

(12) # EUROPEAN PATENT APPLICATION

(21) Application number: 85305096.1

(22) Date of filing: 17.07.85

(51) Int. Cl.[4]: C 07 C 93/10
C 07 C 87/24, C 07 C 119/00
C 07 D 295/02

(30) Priority: 17.07.84 JP 146780/84

(43) Date of publication of application: 05.02.86 Bulletin 86/6

(84) Designated Contracting States: CH DE FR GB LI NL

(71) Applicant: Takasago Perfumery Co., Ltd.
No. 19-22, Takanawa 3-chome
Minato-ku Tokyo(JP)

(72) Inventor: Kumobayashi, Hidenori
1-4-39, Nakakaigan
Chigasaki-shi Kanagawa(JP)

(72) Inventor: Okeda, Yoshiki
1183-6, Kataoka
Hiratsuka-shi Kanagawa(JP)

(72) Inventor: Okazaki, Tetuharu
298-46, Setogaya-cho Hodogaya-ku
Yokohama-shi Kanagawa(JP)

(72) Inventor: Akutagawa, Susumu
1080-22, Shinohara-cho Kohoku-ku
Yokohama-shi Kanagawa(JP)

(74) Representative: Diamond, Bryan Clive et al,
Gee & Co. Chancery House Chancery Lane
London WC2A 1QU(GB)

(54) **Process for the preparation of optically active enamines or imines.**

(57) An allylamine derivative of the formula

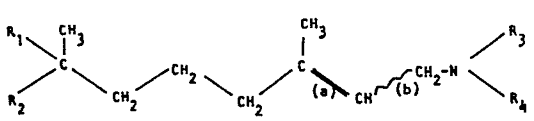

(I)

where $R_1$ = H or $CH_3$, $R_2$ = 4-methyl-pentyl or-$OCH_3$, $R_3$, $R_4$ = 1-4C alkyl or 5-8C cycloalkyl and $R_3$ can be H, and $R_3 + R_4$ can link to form a 5- or 6-numbered ring, is treated with rhodium-phosphine complex as catalyst, of the formula $[Rh(Y)L]^+ X^-$ where Y = ethylene, 1,3-butadiene, cycloxadiene, cycloactadiene or L; X= $ClO_4$, $BF_4$ or $PF_6$, and L is a binaphthyl derivative of formula

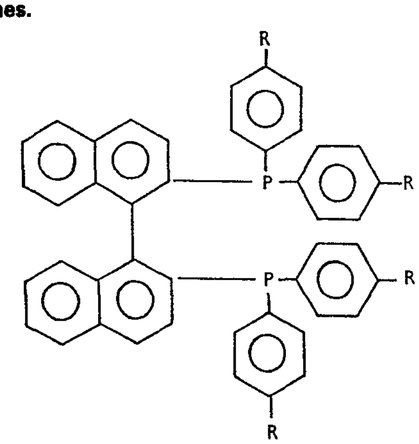

(V)

where R = H, $-CH_3$ or tert-butyl, so as to isomerise the double bond (a) to the position (b) to form the corresponding enamine; if $R_3$ = H, hydrotropy tautomerism occurs at the $CH_2-N-Y-$group to form the imine.

./...

EP 0 170 470 A2

The preparation of the catalyst is described.

The reaction is preferably carried out in a solvent such as an ether, ketone or halide at 80° to 120°C for 1-15 hours using 1:2,000 to 1:10,000 mole catalyst per mole of derivative (I), followed by distillation off of the solvent.

The products are obtained in high yield and of high optically active purity.

# PROCESS FOR THE PREPARATION OF OPTICALLY ACTIVE ENAMINES OR IMINES

The present invention relates to a process for the preparation of optically active enamines or imines. More particularly, it is concerned with an improved catalyst for use in the preparation of optically active enamines or imines by isomerization of allylamine derivatives.

Various methods have been known for the preparation of enamines or imines by the isomerization of allylamine derivatives: a method in which strong bases are used as catalysts (H. Sauer et al., Chem. Ber., 102, 1917 (1969)), a method in which metal oxides are used as catalysts (Tanabe et al., Chem. Lett., 1465 (1977)), a method in which cobalt complexes are used as catalysts (Japanese Patent Publication Nos. 26894/83 and 17447/83), a method in which palladium complexes are used as catalysts (Japanese Patent Publication No. 26893/83), and a method in which rhodium complexes are used as catalysts (Japanese Patent Application (OPI) No. 4748/83). The term "OPI" is used herein to mean a "published unexamined Japanese patent application".

These methods are mainly/of the type of a general isomerization reaction which uses a catalyst and investigations have are not much been made on the asymmetric isomerization reaction. One of the methods for the asymmetric isomerization reaction is described in Japanese Patent Publication No. 17447/83, in which geranylamine derivatives (trans-form) and nerylamine derivatives (cis-form) are isomerized in the presence of optically active cobalt/phosphine complexes to prepare optically active enamines or imines. In this method, the optical purity of the aldehyde thus prepared is as low as 45% or less. It has therefore been desired to develop industrially favorable catalysts for asymmetric isomerization and a method using such catalyst.

As a result of extensive investigations on asymmetric isomerization, we have found that when allylamine derivatives are isomerized in the presence of a specific rhodium/phosphine complex having optically active 2,2'-bis(diphenylphosphino)-1,1'-binaphthyl (hereinafter referred to as "BINAP" for brevity) as a ligand, the corresponding optically active enamines or imines can be obtained in a high purity and also in high yield.

The present invention provides a process of preparing an enamine represented by the formula (II) or imine represented by the formula (III) by isomerizing an allylamine derivative represented by the formula (I) in the presence of a rhodium/phosphine complex represented by the formula (IV). The formulae (I), (II),

(III), and (IV) are described hereinafter.

## DETAILED DESCRIPTION OF THE INVENTION

### Starting Material

The starting material which is used in the process of the present invention is an allylamine derivative represented by the formula (I):

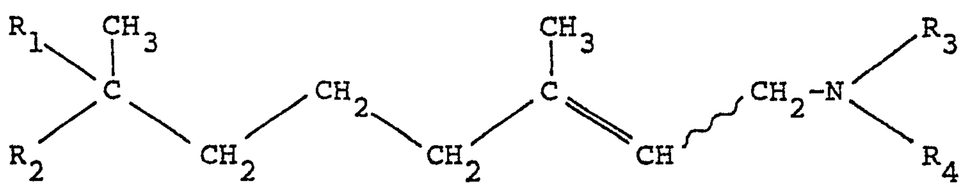

(I)

wherein $R_1$ is hydrogen or a methyl group, $R_2$ is a 4-methylpentyl group or a methoxy group, with the proviso that when $R_1$ is hydrogen, $R_2$ is a 4-methylpentyl group, and when $R_1$ is a methyl group, $R_2$ is a methoxy group, $R_3$ is hydrogen, an alkyl group having from 1 to 4 carbon atoms or a cycloalkyl group having from 5 to 8 carbon atoms, $R_4$ is an alkyl having from 1 to 4 carbon atoms or a cycloalkyl group having from 5 to 8 carbon atoms, and $R_3$ and $R_4$ may link together with the adjacent nitrogen atom form a 5- or 6-membered ring, or with an oxygen atom to form a 6-membered ring.

Representative examples of the allylamine derivatives of the formula (I) include N,N-dimethyl-7-(R,S)-2(E)-3,7,11-trimethyl-2-dodecenylamine, N,N-diethyl-7-(R,S)-2(E)-3,7,11-trimethyl-2-dodecenylamine, N,N-dipropyl-7-(R,S)-2(E)-3,7,11-trimethyl-2-dodecenylamine, N,N-dibutyl-7-(R,S)-2(E)-3,7,11-trimethyl-2-dodecenylamine, N,N-dimethyl-7-(R)-2(E)-3,7,11-trimethyl-2-dodecenylamine, N,N-diethyl-7-(R)-2(E)-3,7,11-trimethyl-2-dodecenylamine, N,N-dimethyl-7-(S)-2(E)-3,7,11-trimethyl-2-dodecenylamine, N,N-diethyl-7-(S)-2(E)-3,7,11-trimethyl-2-dodecenylamine, N,N-dimethyl-7-(S)-2(Z)-3,7,11-trimethyl-2-dodecenylamine, N,N-diethyl-7-(S)-2(Z)-3,7,11-trimethyl-2-dodecenylamine, N,N-dimethyl-7-(R)-2(Z)-3,7,11-trimethyl-2-dodecenylamine, N,N-diethyl-7-(R)-2(Z)-3,7,11-trimethyl-2-dodecenylamine, N-cyclohexyl-7-(R)-2(E)-3,7,11-trimethyl-2-dodecenylamine, 7-(R)-2(E)-3,7,11-trimethyl-2-dodecenyl-1-morpholine, 2(E)-N,N-diethyl-7-methoxy-3,7-dimethyl-2-octenylamine (hereinafter referred to as "N,N-diethyl-7-methoxy-geranylamine"), 2(E)-N,N-dimethyl-7-methoxy-3,7-dimethyl-2-octenylamine, 2(Z)-N,N-dimethyl-7-methoxy-3,7-dimethyl-2-octenylamine, 2(Z)-N,N-diethyl-7-methoxy-3,7-dimethyl-2-octenylamine (hereinafter referred to as "N,N-diethyl-7-methoxy-nerylamine), 2(E)-N-cyclohexyl-7-methoxy-3,7-dimethyl-2-octenylamine (hereinafter referred to as "N-cyclohexyl-7-methoxy-geranylamine), 2(E)-1-morpholino-7-methoxy-3,7-dimethyl-2-octenylamine, 2(E)-1-pyrrolidinyl-7-methoxy-3,7-dimethyl-2-octenylamine, 2(E)-1-piperidino-7-methoxy-3,7-dimethyl-2-octenylamine, and 2(E)-N-butyl-7-methoxy-3,7-dimethyl-2-octenylamine.

Catalyst

The catalyst which is used in the isomerization of the starting material, allylamine derivative, is a rhodium/phosphine complex represented by the formula (IV):

$$[Rh(Y)L]^{+} X^{-} \qquad (IV)$$

wherein Y is ethylene, 1,3-butadiene, cyclohexadiene, cyclooctadiene, or L, X is $ClO_4$, $BF_4$, or $PF_6$, and L is a phosphino binaphthyl derivative represented by the formula (V):

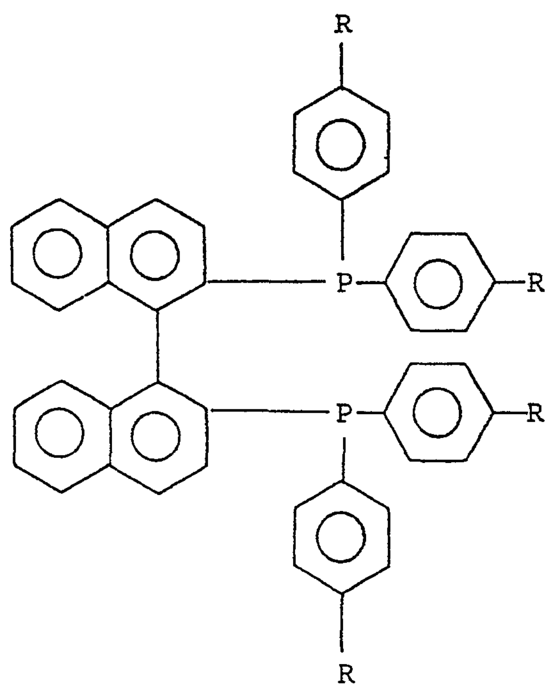

(V)

wherein R is hydrogen, a methyl group, or a tert-butyl group.

The rhodium/phosphine complex of the formula (IV) can be prepared by the methods described in Japanese Patent Application (OPI) No. 20294/84, and European Patent Applications Nos. 84306292.8 (filed September 14, 1984) and 85301846.3 (filed March 15, 1985).

For example, $[Rh(COD)((+)\text{-}BINAP)]^{+}C\ell O_4^{-}$ is prepared by reacting dichlorobis(cycloocta-1,5-diene)rhodium $[RhCl\text{-}(COD)]_2$ and (+)BINAP in a solvent by adding an aqueous solution of sodium perchlorate and laurylbutyl phosphonium bromide.

$[Rh((+)\text{-}BINAP)_2]^{+}C\ell O_4^{-}$ is prepared by adding BINAP to $[Rh(cycloocta\text{-}1,5\text{-}diene)(BINAP)]^{+}C\ell O_4^{-}$ in a solvent and then hydrogenating it.

$[Rh(COD)((+)\ p\text{-}Tolyl\text{-}BINAP)]^{+}C\ell O_4^{-}$ is prepared by reacting (+)p-Tolyl-BINAP with a complex prepared from rhodium trichloride and cycloocta-1,5-diene, in a solvent.

Phosphino binaphthyl derivative of the formula (V) is used as the ligand. Examples thereof include 2,2'-bis-(diphenylphosphino)-1,1'-binaphthyl, 2,2'-bis(di-paratolyl-phosphinyl)-1,1'-binaphthyl (hereinafter referred to as "p-tolyl-BINAP"), and 2,2'-bis(di-para-tert-butylphosphinyl)-1,1'-binaphthyl (hereinafter referred to as "t-butyl BINAP").

Isomerization

The starting material, allylamine derivative, of the

formula (I) is isomerized in a solvent at 80 to 120°C for 1 to 15 hours in the presence of the rhodium/phosphine complex of the formula (IV). The amount of the rhodium/phosphine complex catalyst used is from 1/2,000 to 1/10,000 mole per mole of the allylamine derivative. Solvents which can be used include ethers such as tetrahydrofuran, ketones such as acetone and halides such as methylene chloride. In conducting the isomerization, it is preferred that the rhodium/phosphine complex catalyst be added to the above solvent and the resulting uniform solution is used. The amount of the solvent used is preferably from 1/2 to 4 times (by volume) that of the allylamine derivative.

After the reaction is completed, the solvent is distilled away by conventional techniques and then the desired optically active enamine or imine is obtained by distillation.

Desired Product

When an allylamine derivative of the formula (I) in which $R_3$ is a substituent other than hydrogen is used, an enamine is obtained. On the other hand, when an allylamine in which $R_3$ is hydrogen is used, the product undergoes so-called hydrotropy tautomerism, resulting in the formation of an imine in a stable form.

The enamines prepared by the process of the present invention are represented by the formula (II):

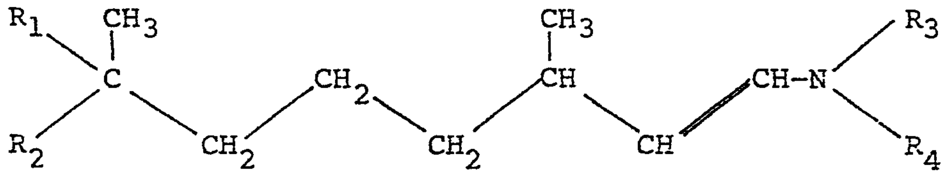 (II)

wherein $R_1$, $R_2$, $R_3$, and $R_4$ are the same as defined above.

The imines are represented by the formula (III):

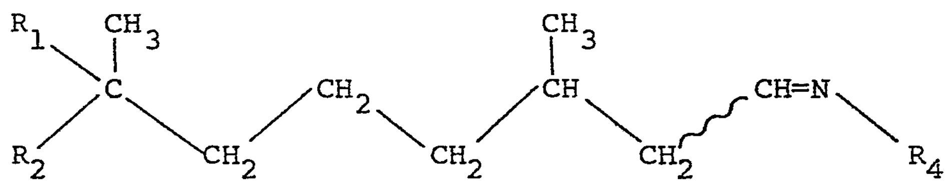 (III)

wherein $R_1$, $R_2$, and $R_4$ are the same as defined above.

The process of the present invention has various advantages. For example, the use of a small amount of the specific rhodium/phosphine complex catalyst permits asymmetric isomerization of various allylamines in high yield. The optically active enamines and imines thus prepared have a high optical purity.

These enamines and imines are quantitatively hydrolyzed, thereby producing the corresponding aldehydes or ketones. The optically active aldehydes thus prepared are very important as intermediates for the production of perfumes, medicines such as α-tocopherol, agricultural chemicals such as juvenile hormones, and so forth.

The present invention is described in greater detail

by reference to the following examples. All percentages are by weight.

The optical rotation of the phosphino binaphthyl derivatives used are shown below.

| | $[\alpha]_D^{25}$ | |
|---|---|---|
| (+)BINAP | +225° | (C=0.7, benzene) |
| (-)BINAP | -224° | (C=1.0, benzene) |
| (+)p-tolyl-BINAP | +170° | ( " , " ) |
| (-)p-tolyl-BINAP | -170.5° | ( " , " ) |
| (+)tert-butyl-BINAP | +83.2° | ( " , " ) |
| (-)tert-butyl-BINAP | -83.0° | ( " , " ) |

EXAMPLE 1

A pressure reactor, the atmosphere of which had been replaced with nitrogen, was charged with 93.2 mg (0.1 mmol) of $[Rh(COD)(+)BINAP]^+ClO_4^-$ and 200 ml of tetrahydrofuran, and finally with 193 g (0.8 mol) of N,N-diethyl-7-methoxygeranylamine. The resulting mixture was reacted at 100°C for 5 hours. After completion of the reaction, the tetrahydrofuran was distilled away and the reaction product was distilled in a vacuum of 3.5 mmHg to obtain 190 g of a fraction having a boiling point of 105 to 106°C. GLC analysis showed that the fraction was a mixture of 2% of

N,N-diethyl-7-methoxy-geranylamine and 98% of N,N-diethyl-7-methoxy-3,7-dimethyl-1-octenylamine.

In order to determine the optical purity of the enamine prepared by the above isomerization reaction, the above fraction was dissolved in 300 ml of toluene, and a 10% aqueous solution of sulfuric acid was added dropwise with stirring while maintaining the temperature at 0°C. When the pH of the solution reached 4 to 5, the addition of the aqueous sulfuric acid was stopped, and the resulting mixture was heated to room temperature with stirring and then allowed to separate.

The toluene layer was washed twice with 300 ml of water and then with 300 ml of a saturated aqueous solution of sodium bicarbonate. After separation, the toluene layer was dried with anhydrous magnesium sulfate. The toluene was distilled away in vacuum and the residue was vacuum distilled to obtain 141 g of a fraction having a boiling point of 99 to 100°C/6 mmHg. GLC and NMR analysis showed that the product was 3-(S)-3,7-dimethyl-7-methoxyoctanal (purity: 99.5%). The optical rotation $[\alpha]_D^{25}$ was -12.22° ($[\alpha]_D^{25}$ = -12.13° in the literature) and its optical purity was substantially 100%.

EXAMPLE 2

The procedure of Example 1 was repeated except that 145 mg (0.1 mmol) of $[Rh((+)BINAP)_2]^+C\ell O_4^-$ and 200 ml of

tetrahydrofuran, and then 193 g (0.8 mol) of N,N-diethyl-7-methoxy-geranylamine were added, and the reaction was conducted at 100°C for 15 hours, to obtain 191 g of a fraction having a boiling point of 106 to 106.5°C/4 mmHg. GLC analysis showed that the fraction was a mixture of 1.5% of N,N-diethyl-7-methoxy-geranylamine and 98.5% of N,N-diethyl-7-methoxy-3,7-dimethyl-1-octenylamine.

The optical rotation of the enamine as determined in the same manner as in Example 1 was $[\alpha]_D^{25} = -12.2°$.

EXAMPLE 3

The procedure of Example 1 was repeated except that 99 mg (0.1 mmol) of $[Rh(COD)(+)p\text{-tolyl-BINAP}]^+ClO_4^-$ and 300 ml of tetrahydrofuran, and then 193 g (0.8 mol) of N,N-diethyl-7-methoxy-geranylamine were added, and the reaction was conducted at 100°C for 3 hours, to obtain 189 g of a fraction having a boiling point of 106°C/4 mmHg. GLC analysis showed that the fraction was a mixture of 5% of N,N-diethyl-7-methoxy-geranylamine and 95% of N,N-diethyl-7-methoxy-3,7-dimethyl-1-octenylamine.

The optical rotation of the aldehyde derived in the same manner as in Example 1 was $[\alpha]_D^{25} = -12.13°$.

EXAMPLE 4

The procedure of Example 1 was repeated except that 116 mg (0.1 mmol) of $[Rh(COD)((-)\text{-butyl-BINAP})]^+ClO_4^-$ and 250 ml of tetrahydrofuran, and then 193 g (0.8 mol) of N,N-diethyl-7-methoxy-nerylamine were added, and the reaction was conducted at 100°C for 10 hours, to obtain 190 g of a fraction having a boiling point of 105°C/4 mmHg. GLC analysis showed that the fraction was a mixture of 3% of N,N-diethyl-7-ethoxy-nerylamine and 97% of N,N-diethyl-7-methoxy-3,7-dimethyl-1-octenylamine.

The optical rotation of the aldehyde derived in the same manner as in Example 1 was $[\alpha]_D^{25} = -11.96°$.

EXAMPLE 5

The procedure of Example 1 was repeated except that 156 mg (0.1 mmol) of $[Rh((-)p\text{-tolyl-BINAP})_2]^+ClO_4^-$ and 200 ml of tetrahydrofuran, and then 193 g (0.8 mol) of N,N-diethyl-7-methoxy-geranylamine were added, and the reaction was conducted at 110°C for 10 hours, to obtain 191 g of a fraction having a boiling point of 106°C/4 mmHg. GLC analysis showed that the fraction was a mixture of 2.3% of N,N-diethyl-7-methoxy-geranylamine and 97.7% of N,N-diethyl-7-methoxy-3,7-dimethyl-1-octenylamine.

The optical rotation of the aldehyde derived in the same manner as in Example 1 was $[\alpha]_D^{25} = +12.18°$.

EXAMPLE 6

A pressure reactor, the atmosphere of which had been replaced with nitrogen, was charged with 103 mg (0.1 mmol) of $[Rh(COD)((-)p\text{-tolyl-BINAP})]^+PF_6^-$ and 200 ml of tetrahydrofuran, and then 56 g (0.2 mol) of N,N-diethyl-7-(R)-2-

(E)-3,7,11-trimethyl-2-dodecenylamine. The reaction mixture was reacted at 100°C for 8 hours. After completion of the reaction, the tetrahydrofuran was distilled away in a vacuum, and the residue was vacuum distilled to obtain 53 g of a fraction having a boiling point of 95 to 100°C/0.5 mmHg. GLC analysis showed that the fraction was a mixture of 2% of unreacted amine and 98% of N,N-diethyl-7-(R)-3,7,11-trimethyl-1-dodecenylamine.

In the same manner as in Example 1, an aldehyde having a boiling point of 100 to 105°C/0.8 mmHg was derived from the fraction, and this aldehyde was oxidized with silver oxide to form 3,7,11-trimethyldodecanic acid. The optical rotation of the 3,7,11-trimethyldodecanic acid was measured and found to be $[\alpha]_D^{25} = +5.38°$. The optical purity of a newly formed asymmetric carbon atom in the 3-position was about 99%.

EXAMPLE 7

The procedure of Example 6 was repeated except that 145 mg (0.1 mmol) of $[Rh((-)\text{-BINAP})_2]^+ClO_4^-$, 300 ml of tetrahydrofuran, and 56 g (0.2 mol) of N,N-diethyl-7-(R)-2-(E)-3,7,11-trimethyl-2-dodecenylamine were added, and the reaction was conducted at 100°C for 15 hours, to obtain 51 g of N,N-diethyl-7-(R)-3,7,11-trimethyl-1-dodecenylamine (containing 5% of unreacted amine). The optical rotation of a carboxylic acid derived in the same manner as in Example 6

was measured and found to be $[\alpha]_D^{25}$ = +5.35°. The optical purity of a newly formed asymmetric carbon atom in the 3-position was 98.5%.

EXAMPLE 8

The procedure of Example 6 was repeated except that 116 mg (0.1 mmol) of $[Rh(COD)((+)tert\text{-}butyl\ BINAP)]^+ClO_4^-$, 200 ml of tetrahydrofuran, and 56 g (0.2 mol) of N,N-diethyl-7-(R)-2-(Z)-3,7,11-trimethyl-2-dodecenylamine were added, and the reaction was conducted at 110°C for 8 hours. The enamine thus prepared was converted into a carboxylic acid in the same manner as in Example 6, and the optical rotation of the carboxylic acid was measured and found to be $[\alpha]_D^{25}$ = +5.27°. The optical purity of a newly formed asymmetric carbon atom in the 3-position was about 97.1%.

EXAMPLE 9

The procedure of Example 6 was repeated except that 114.4 mg (0.1 mmol) of $[Rh(COD)((-)tert\text{-}butyl\ BINAP)]^+BF_6^-$, 200 ml of tetrahydrofuran, and 56 g (0.2 mol) of N,N-diethyl-7-(RS)-2-(E)-3,7,11-trimethyl-2-dodecenylamine were added, and the reaction was conducted at 100°C for 15 hours. The enamine thus formed was converted into a carboxylic acid in the same manner as in Example 1, and the optical rotation of the carboxylic acid was measured and found to be $[\alpha]_D^{25}$ = +5.86°. The optical purity of a newly formed asymmetric carbon atom in the 3-position was about 97%.

EXAMPLE 10

The procedure of Example 1 was repeated except that 93 mg (0.1 mmol) of $[Rh(COD)((+)-BINAP)]^{+}C\ell O_4^{-}$ and 200 ml of tetrahydrofuran, and then 213 g (0.69 mol) of N-cyclohexyl-7-methoxy-geranylamine were added, and the reaction was conducted at 90°C for 8 hours, to obtain 202 g of a fraction having a boiling point of 110 to 113°C/1.2 mmHg. GLC analysis showed that the fraction was a mixture of 1.2% of unreacted amine and 98.8% of 7-methoxy-3,7-dimethyl-1-octanal-cyclohexylimine. 7-Methoxy-3,7-dimethyl-1-octanal derived in the same manner as in Example 1 had an optical rotation of $[\alpha]_D^{25} = -11.8°$.

EXAMPLE 11

The procedure of Example 6 was repeated except that 93 mg (0.1 mmol) of $[Rh(COD)((-)p\text{-tolyl-BINAP})]^{+}C\ell O_4^{-}$, 250 ml of tetrahydrofuran, and 59 g (0.2 mol) of 7-(R)-2-(E)-3,7,11-trimethyl-2-dodecenyl-1-morpholine were added, and the reaction was conducted at 100°C for 10 hours. The optical purity of a newly formed asymmetric carbon atom in the 3-position was about 95%.

CLAIMS:

1. A process for the preparation of an enamine represented by the formula (II):

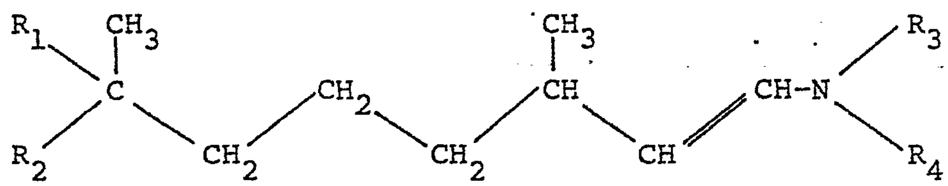

(II)

wherein $R_1$ is hydrogen or a methyl group, $R_2$ is a 4-methylpentyl group or a methoxy group, with the proviso that when $R_1$ is hydrogen, $R_2$ is a 4-methylpentyl group and when $R_1$ is a methyl group, $R_2$ is a methoxy group, $R_3$ is hydrogen or an alkyl group having from 1 to 4 carbon atoms or a cycloalkyl group having from 5 to 8 carbon atoms; $R_4$ is an alkyl group having from 1 to 4 carbon atoms or a cycloalkyl group having 5 to 8 carbon atoms, and $R_3$ and $R_4$ may link together with the adjacent nitrogen atom to form a 5- or 6-membered ring, or with an oxygen atom to form a 6-membered ring, or of an imine represented by the formula (III):

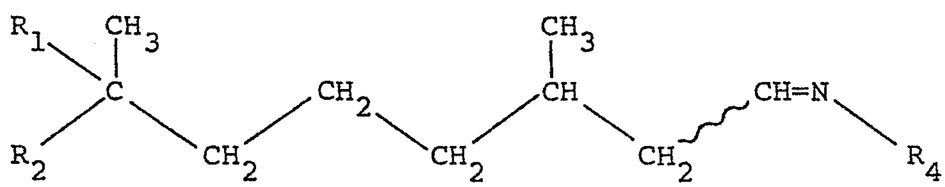

(III)

wherein $R_1$, $R_2$, and $R_4$ are the same as defined above, which

comprises isomerizing an allylamine derivative represented by the formula (I):

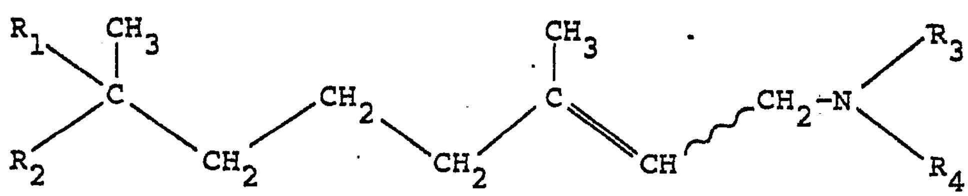

(I)

wherein $R_1$, $R_2$, $R_3$, and $R_4$ are the same as defined above, in the presence of a catalyst, characterised in that the catalyst is a rhodium/phosphine complex represented by the formula (IV):

$$[Rh(Y)L]^+ X^- \quad (IV)$$

wherein Y is ethylene, 1,3-butadiene, cyclohexadiene, cyclooctadiene, or L, X is $C\ell O_4$, $BF_4$, or $PF_6$, and L is a phosphino binaphthyl derivative represented by the formula (V):

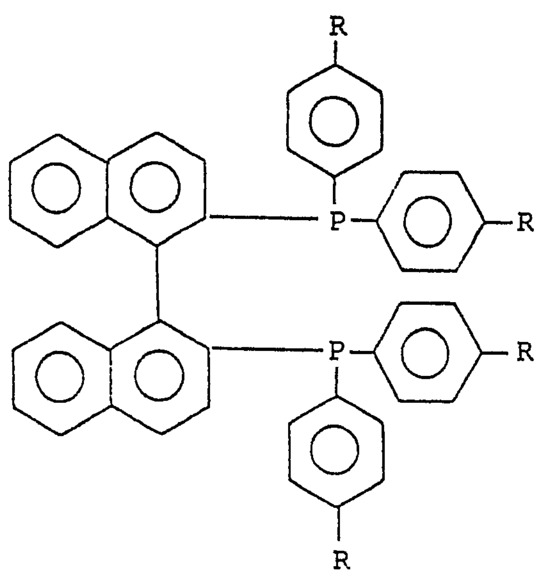

(V)

wherein R is hydrogen, a methyl group, or a tert-butyl group.

2. A process as in Claim 1, wherein the amount of the catalyst used is from 1/2,000 to 1/10,000 mole per mole of the allylamine derivative.

3. A process as in Claim 1 or 2, wherein the isomerization is carried out in a solvent.

4. A process as in Claim 3, wherein the solvent is an ether, ketone or halide.

5. A process as in Claim 3 or 4, wherein the amount of the solvent used is from 1/2 to 4 times by volume that of the allylamine derivative.